# EUROPEAN PATENT APPLICATION

(11) **EP 4 672 250 A2**
(43) Date of publication of application: **31.12.2025**
(21) Application number: 25185176.2
(22) Date of filing: 25.06.2025
(51) Int. Cl.: G16C 20/30, G16C 20/70

(54) **MACHINE LEARNING MODELS FOR HIERARCHICAL MOLECULAR PROPERTY PREDICTION**

(30) Priority: 25.06.2024 GR 20240100459
(71) Applicant: Isomorphic Labs Limited, London EC2M 4RB (GB)
(72) Inventor: KURIN, Vitaly, London, EC2M 4RB (GB); ROSE, Harry, London, EC2M4RB (GB); TIGAS, Panagiotis, London, EC2M4RB (GB); SMITH, Raymond Wensley, London, EC2M 4RB (GB); RICHARDS, Simon James, London, EC2M 4RB (GB); EVANS, Sian Elizabeth, London, EC2M 4RB (GB)
(74) Representative: Marks & Clerk GST

(57) **Abstract**

Methods, systems, and apparatus, including computer programs encoded on a computer storage medium, for predicting properties of molecules based on the structure and composition of the molecules. In one aspect, a method comprises receiving molecule data characterizing a molecule and processing a model input based on the molecule data characterizing the molecule using a property prediction machine learning model comprising an ordered sequence of processing layers to generate, for each of a set of molecule properties, a respective value for the molecule property wherein: each processing layer is associated with a respective proper subset of the set of molecule properties; and each processing layer after the first in the sequence generates predicted molecule property values based on: (i) the model input to the property prediction machine learning model, and (ii) predicted molecule property values generated by one or more preceding processing layers in the sequence of processing layers.

## Description

### BACKGROUND

This specification relates to processing data using machine learning models.

Machine learning models receive an input and generate an output, e.g., a predicted output, based on the received input. Some machine learning models are parametric models and generate the output based on the received input and on values of the parameters of the model.

Some machine learning models are deep models that employ multiple layers of models to generate an output for a received input. For example, a deep neural network is a deep machine learning model that includes an output layer and one or more hidden layers that each apply a nonlinear transformation to a received input to generate an output.

### SUMMARY

This specification generally describes a system implemented as computer programs on one or more computers in one or more locations that can predict properties of molecules based on the structure and composition of the molecules. In particular, the described systems can predict molecule properties following a hierarchy of the molecule properties.

Throughout this specification, an "embedding" of an entity (e.g., a molecule) refers to a representation of the entity as an ordered collection of numerical values, e.g., a vector, matrix, or other tensor of numerical values.

Throughout this specification, a "block" in a neural network refers to a group of one or more neural network layers that are included in the neural network.

Computationally predicting chemical and physical properties of molecules is of significant utility in fields such as drug discovery and development. Accurately predicting values for such molecule properties can aid in determining biological effects of therapeutic agents and can facilitate the development of more effective and selective therapeutic agents. For example, computationally predicted molecule properties for a library of candidate molecules can be used to identify candidate molecules that have desirable characteristics for potential use as therapeutic agents. The system described in this specification can therefore contribute to accelerating the process of drug discovery.

Molecular properties can depend on one another such that predicted values for certain molecular properties can be relevant and useful for predicting values for other molecular properties. For example, a distribution coefficient (log D) of a molecule can depend on a partition coefficient (log P) of the molecule, and therefore a predicted value for the partition coefficient of the molecule can be useful for more accurately predicting a value for the distribution coefficient of the molecule.

The molecular properties can also depend on chemical constraints such that values for certain molecular properties constrain possible values for other molecular properties. As one example, for a neutral molecule and at a neutral acidity, a distribution coefficient (log D) of the molecule can be constrained to be equal to a partition coefficient (log P) of the molecule. As another example, for a charged molecule and at a neutral acidity, a distribution coefficient (log D) of the molecule can be constrained to be smaller than a partition coefficient (log P) of the molecule.

In order to predict molecule properties that can depend on and constrain one another, the described systems can predict molecule properties following a hierarchy of the molecule properties. As an example, in some implementations, the described systems can predict molecule properties following a pre-determined hierarchy (e.g., as determined by an expert) of the molecule properties using sequences of processing layers, with each processing layer being configured to predict a specific subset of the molecule properties associated with the processing layer. As another example, in some implementations, the described systems can predict molecule properties following a machine learned hierarchy of the properties using sequences of self-attention layers, with each self-attention layer being configured to process predicted values for the molecule properties in accordance with learned dependencies between the molecule properties.

The subject matter described in this specification can be implemented in particular embodiments so as to realize one or more of the following advantages.

By predicting molecule properties following a hierarchy of the molecule properties, the described systems can more accurately predict molecule properties that depend on and constrain one another. This can enable the described systems to be trained using less training data and in a shorter training time to reach a desired level of performance (e.g., with respect to prediction accuracy). Compared to conventional methods for molecule property prediction, the described systems can therefore be trained using fewer computational resources (e.g., with respect to processing time, memory consumption, power consumption, data storage, etc.).

In some implementations, the described systems can be trained using an objective function that enforces chemical constraints for the predicted molecule property values, e.g., by penalizing predicted molecule property values that do not follow the chemical constraints. This can enable the described systems to learn to model chemical constraints for molecule properties more efficiently (e.g., using less training data and in a shorter training time) and can therefore further reduce the computational resources (e.g., with respect to processing time, memory consumption, power consumption, data storage, etc.) required to train the described systems.

Additionally, using a hierarchical prediction architecture, the described systems can be easily extended to predict additional molecule properties after training. For example, a property prediction machine learning model can be trained to predict an initial set of molecule properties using the techniques described above. The trained property prediction machine learning model can then be trained to predict additional molecule properties by adding and training additional processing layers, without retraining the entire property prediction machine learning model.

The described systems can be trained to predict molecule properties as conditioned on a data quality for training examples. This enables the described system to be trained using training data of varying data quality while generating predicted molecule property values characteristic of the highest quality training data available. By conditioning prediction based on data quality, the described systems can therefore be trained using a greater variety of training data without sacrificing prediction accuracy.

According to a first aspect, there is provided a method performed by one or more computers, the method comprising: receiving molecule data characterizing a molecule (e.g., a drug candidate); and processing a model input based on the molecule data characterizing the molecule using a property prediction machine learning model to generate, for each molecule property in a set of multiple molecule properties, a respective predicted molecule property value of the molecule property for the molecule, wherein: the property prediction machine learning model comprises an ordered sequence of processing layers; each processing layer in the ordered sequence of processing layers is associated with a respective proper subset of the set of molecule properties; and each processing layer after the first processing layer in the ordered sequence of processing layers generates a respective predicted molecule property value of each molecule property associated with the processing layer based on: (i) the model input to the property prediction machine learning model, and (ii) predicted molecule property values generated by one or more preceding processing layers that precede the processing layer in the sequence of processing layers.

In some implementations, the first processing layer in the ordered sequence of processing layers generates a respective predicted molecule property value of each molecule property associated with the first processing layer based on the model input to the property prediction machine learning model.

In some implementations, for each molecule property in the set of molecule properties, exactly one processing layer of the ordered sequence of processing layers generates a predicted molecule property value for the molecule property.

In some implementations, the property prediction machine learning model has been trained by operations comprising: obtaining training data comprising a plurality of training examples, wherein each training example corresponds to a respective training molecule and includes: (i) a training model input that comprises data characterizing the training molecule, and (ii) a target molecule property value for the training molecule for each of one or more molecule properties of the set of multiple molecule properties; and training the property prediction machine learning model on the plurality of training examples using a machine learning technique.

In some implementations, training the property prediction machine learning model on the plurality of training examples using the machine learning technique comprises, for each of the plurality of training examples: processing the training model input of the training example using the property prediction machine learning model and in accordance with current values of a set of property prediction machine learning model parameters to generate, for each molecule property in the set of multiple molecule properties, a respective predicted molecule property value for the corresponding training molecule; and updating the current values of the set of property prediction machine learning model parameters based on an objective function that depends on the predicted molecule property values generated by the property prediction machine learning model for the training example.

In some implementations, the objective function includes a term that measures a discrepancy between: (i) the target molecule property values specified by the training example, and (ii) the predicted molecule property values generated by the property prediction machine learning model by processing the training model input of the training example.

In some implementations, the objective function includes a term that evaluates whether the predicted molecule property values generated by the property prediction machine learning model satisfy a chemical constraint on molecule property values.

In some implementations, the chemical constraint of molecule property values defines, for a plurality of molecule properties, an allowable region in a joint space of possible values of the plurality of molecule properties; and the chemical constraint penalizes predicted molecule property values for the plurality of molecule properties that are outside the allowable region in the joint space of possible values of the plurality of molecule properties.

In some implementations, the chemical constraint requires that, for a neutral molecule, a logarithm of a partition coefficient (log P) of the molecule is equal to a logarithm of a distribution coefficient (log D) of the molecule at a neutral power of hydrogen (pH).

In some implementations, the chemical constraint requires that, for a charged molecule, a logarithm of a partition coefficient (log P) of the molecule is greater than a logarithm of a distribution coefficient (log D) of the molecule at a neutral power of hydrogen (pH).

In some implementations, for each training example and for each of the set of multiple molecule properties, the target molecule property value for the molecule of the training example is a molecule property value for the molecule of the training example under a shared set of experimental conditions.

In some implementations, processing the model input based on the molecule data characterizing the molecule using the property prediction machine learning model to generate, for each molecule property in a set of multiple molecule properties, a respective predicted molecule property value of the molecule property for the molecule comprises: processing the model input based on the molecule data characterizing the molecule using an embedding neural network to generate an embedding of the molecule; and wherein for each processing layer after the first processing layer in the ordered sequence of processing layers of the property prediction machine learning model, the processing layer processes a layer input that comprises: (i) the embedding of the molecule generated by the embedding neural network, and (ii) the predicted molecule property values generated by the one or more preceding processing layers that precede the processing layer in the sequence of processing layers.

In some implementations, the model input comprises a graph characterizing the molecule; and the embedding neural network comprises a graph neural network.

In some implementations, the graph characterizing the molecule includes a plurality of graph nodes, wherein each graph node characterizes a corresponding atom within the molecule.

In some implementations, each graph node is associated with a respective atom embedding that includes data characterizing properties of the corresponding atom of the molecule.

In some implementations, the graph includes one or more graph edges, wherein each graph edge connects a respective pair of graph nodes within the graph and characterizes a corresponding chemical bond between a pair of atoms within the molecule.

In some implementations, each graph edge is associated with a respective bond embedding that includes data characterizing properties of the corresponding chemical bond of the molecule.

In some implementations, the graph neural network comprises one or more update layers.

In some implementations, processing the model input based on the molecule data characterizing the molecule using the embedding neural network to generate the embedding of the molecule: for each of one or more update iterations, updating the embeddings associated with the graph nodes and graph edges by processing the model input using the one or more update layers of the graph neural network; and generating the embedding of the molecule based on the updated embeddings associated with the graph nodes and graph edges.

In some implementations, updating the embeddings associated with the graph nodes and graph edges comprises: for each graph node of the graph, updating the embedding associated with the graph node based on the embeddings associated with neighboring graph nodes that are connected to the graph node by respective graph edges; and for each graph edge of the graph, updating the embedding associated with the graph edge based on the embeddings associated with the graph nodes connected by the graph edge.

In some implementations, generating the embedding of the molecule based on the updated embeddings associated with the graph nodes and graph edges comprises generating the embedding of the molecule based on a combination of the updated embeddings associated with the graph nodes.

In some implementations, generating the embedding of the molecule based on a combination of the updated embeddings associated with the graph nodes comprises generating the embedding of the molecule based on a summation of the updated embeddings associated with the graph nodes.

In some implementations, the set of multiple molecule properties for the molecule includes a partition coefficient (log P) for the molecule.

In some implementations, the set of multiple molecule properties for the molecule includes a distribution coefficient (log D) for the molecule.

In some implementations, the set of multiple molecule properties includes a classification of whether the molecule is acidic.

In some implementations, the set of multiple molecule properties includes a classification of whether the molecule is basic.

In some implementations, for each training example, the training model input for the training example includes data characterizing a data quality of the target molecule property values for the training example.

In some implementations, the model input is based on the molecule data characterizing the molecule includes data characterizing a particular data quality.

In some implementations, the model input is based on the molecule data characterizing the molecule includes data characterizing a highest data quality.

In some implementations, the property prediction machine learning model is one of an ensemble of property prediction machine learning models.

In some implementations, the method further comprises: processing the model input based on the molecule data characterizing the molecule using one or more additional property prediction machine learning models from the ensemble of property prediction machine learning models to determine, for each molecule property in the set of multiple molecule properties, a respective distribution of molecule property values of the molecule property for the molecule.

According to another aspect, there is provided a method performed by one or more computers, the method comprising: receiving molecule data characterizing a molecule; processing a model input based on the molecule data characterizing the molecule using a property prediction neural network to generate, for each molecule property in a set of multiple molecule properties, a respective predicted molecule property value of the molecule property for the molecule, comprising: processing the model input using a molecule embedding block of the property prediction neural network to generate an embedding of the molecule; generating a respective property-specific embedding for each molecule property in the set of multiple molecule properties based on the embedding of the molecule; processing the property-specific embeddings of the molecule properties in the set of molecule properties by applying one or more self-attention operations to update the property-specific embeddings of the molecule properties; and after applying the self-attention operations to the property-specific embeddings of the molecule properties, processing the property-specific embeddings of the molecule properties to generate the predicted molecule property values.

In some implementations, the property prediction neural network comprises one or more self-attention layers; and processing the property-specific embeddings of the molecule properties in the set of molecule properties by applying one or more self-attention operations to update the property-specific embeddings of the molecule properties comprises, for each of the one or more self-attention layers of the property prediction neural network: processing, by the self-attention layer, the property-specific embeddings of the molecule properties to generate a respective attention weight for each pair of property-specific embeddings of the molecule properties; and updating, by the self-attention layer, the property-specific embeddings of the molecule properties based on the attention weights generated by the self-attention layer.

In some implementations, the property prediction neural network has been trained by operations comprising: obtaining training data comprising a plurality of training examples, wherein each training example corresponds to a respective training molecule and includes: (i) a training model input that comprises data characterizing the training molecule, and (ii) a target molecule property value for the training molecule for each of one or more molecule properties of the set of multiple molecule properties; and training the property prediction neural network on the plurality of training examples using a machine learning technique.

In some implementations, training the property prediction neural network on the plurality of training examples using the machine learning technique comprises, for each of the plurality of training examples: processing the training model input of the training example using the property prediction neural network and in accordance with current values of a set of property prediction neural network parameters to generate, for each molecule property in the set of multiple molecule properties, a respective predicted molecule property value for the corresponding training molecule; and updating the current values of the set of property prediction neural network parameters based on an objective function that depends on the predicted molecule property values generated by the property prediction neural network for the training example.

In some implementations, the objective function includes a term that measures a discrepancy between: (i) the target molecule property values specified by the training example, and (ii) the predicted molecule property values generated by the property prediction neural network by processing the training model input of the training example.

In some implementations, the objective function includes a term that evaluates whether the predicted molecule property values generated by the property prediction neural network satisfy a chemical constraint on molecule property values.

In some implementations, the chemical constraint of molecule property values defines, for a plurality of molecule properties, an allowable region in a joint space of possible values of the plurality of molecule properties; and the chemical constraint penalizes a predicted molecule property values for the plurality of molecule properties that are outside the allowable region in the joint space of possible values of the plurality of molecule properties.

In some implementations, the chemical constraint requires that, for a neutral molecule, a logarithm of a partition coefficient (log P) of the molecule is equal to a logarithm of a distribution coefficient (log D) of the molecule at a neutral power of hydrogen (pH).

In some implementations, the chemical constraint requires that, for a charged molecule, a logarithm of a partition coefficient (log P) of the molecule is greater than a logarithm of a distribution coefficient (log D) of the molecule at a neutral power of hydrogen (pH).

In some implementations, for each training example and for each of the set of multiple molecule properties, the target molecule property value for the molecule of the training example is a molecule property value for the molecule of the training example under a shared set of experimental conditions.

In some implementations, the model input comprises a graph characterizing the molecule; and the molecule embedding block of the property prediction neural network comprises a graph neural network.

In some implementations, the graph characterizing the molecule includes a plurality of graph nodes, wherein each graph node characterizes a corresponding atom within the molecule.

In some implementations, each graph node is associated with a respective atom embedding that includes data characterizing properties of the corresponding atom of the molecule.

In some implementations, the graph includes one or more graph edges, wherein each graph edge connects a respective pair of graph nodes within the graph and characterizes a corresponding chemical bond between a pair of atoms within the molecule.

In some implementations, each graph edge is associated with a respective bond embedding that includes data characterizing properties of the corresponding chemical bond of the molecule.

In some implementations, the graph neural network comprises one or more update layers.

In some implementations, processing the model input using the molecule embedding block of the property prediction neural network to generate the embedding of the molecule comprises: for each of one or more update iterations, updating the embeddings associated with the graph nodes and graph edges by processing the model input using the one or more update layers of the graph neural network; and generating the embedding of the molecule based on the updated embeddings associated with the graph nodes and graph edges.

In some implementations, updating the embeddings associated with the graph nodes and graph edges comprises: for each graph node of the graph, updating the embedding associated with the graph node based on the embeddings associated with neighboring graph nodes that are connected to the graph node by respective graph edges; and for each graph edge of the graph, updating the embedding associated with the graph edge based on the embeddings associated with the graph nodes connected by the graph edge.

In some implementations, generating the embedding of the molecule based on the updated embeddings associated with the graph nodes and graph edges comprises generating the embedding of the molecule based on a combination of the updated embeddings associated with the graph nodes.

In some implementations, generating the embedding of the molecule based on a combination of the updated embeddings associated with the graph nodes comprises generating the embedding of the molecule based on a summation of the updated embeddings associated with the graph nodes.

In some implementations, the set of multiple molecule properties for the molecule includes a partition coefficient (log P) for the molecule.

In some implementations, the set of multiple molecule properties for the molecule includes a distribution coefficient (log D) for the molecule.

In some implementations, the set of multiple molecule properties includes a classification of whether the molecule is acidic.

In some implementations, the set of multiple molecule properties includes a classification of whether the molecule is basic.

In some implementations, for each training example, the training model input for the training example includes data characterizing a data quality of the target molecule property values for the training example.

In some implementations, the model input is based on the molecule data characterizing the molecule includes data characterizing a particular data quality.

In some implementations, the model input is based on the molecule data characterizing the molecule includes data characterizing a highest data quality.

In some implementations, the property prediction neural network is one of an ensemble of property prediction neural networks.

In some implementations, the method further comprises processing the model input is based on the molecule data characterizing the molecule using one or more additional property prediction neural networks from the ensemble of property prediction neural networks to determine, for each molecule property in the set of multiple molecule properties, a respective distribution of molecule property values of the molecule property for the molecule.

According to another aspect, there is provided a method, comprising: receiving data identifying a collection of molecules; generating, for each molecule in the collection of molecules, predicted molecule property values for each of a set of multiple molecule properties of the molecule using the methods described herein; and selecting one or more molecules in the collection of molecules for physical synthesis based, at least in part, on the predicted molecule property values.

In some implementations, selecting the one or more molecules in the collection of molecules for physical synthesis based, at least in part, on the predicted molecule property values comprises, for each molecule in the collection of molecules: evaluating whether the molecule satisfies one or more criteria based on the predicted molecule property values; and determining whether to select the molecule for physical synthesis based on whether the molecule satisfies the one or more criteria based on the predicted molecule property values.

In some implementations, the method further comprises physically synthesizing one or more of the molecules selected for physical synthesis.

In some implementations, the method further comprises, for each of one or more of the molecules selected for physical synthesis: performing a physical experiment using a synthesized instance of the molecule to determine experimental values for each of one or more experimentally validated molecule properties of the molecule.

In some implementations, the one or more experimentally validated molecule properties includes a partition coefficient (log P) for the molecule.

In some implementations, the one or more experimentally validated molecule properties for the molecule includes a distribution coefficient (log D) for the molecule.

In some implementations, the one or more experimentally validated molecule properties includes a classification of whether the molecule is acidic.

In some implementations, the one or more experimentally validated molecule properties includes a classification of whether the molecule is basic.

According to another aspect there is provided a system comprising: one or more computers; and one or more storage devices communicatively coupled to the one or more computers, wherein the one or more storage devices store instructions that, when executed by the one or more computers, cause the one or more computers to perform the operations of the methods described herein.

According to another aspect, there are provided one or more non-transitory computer storage media storing instructions that when executed by one or more computers cause the one or more computers to perform the operations of the methods described herein.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 is a block diagram of an example molecule property prediction system.
FIG. 2 is a block diagram of an example property prediction machine learning model.
FIG. 3 illustrates training a property prediction machine learning model.
FIG. 4 is a flow diagram of an example process for predicting molecule property values.
FIG. 5A is a flow diagram of an example process for predicting molecule property values using a property prediction machine learning model with a sequence of processing layers.
FIG. 5B illustrates an example of predicting molecule property values using a property prediction machine learning model with a sequence of processing layers.
FIG. 6 is a flow diagram of an example process for predicting molecule property values by performing self-attention operations using a property prediction machine learning model.
FIG. 7A is a flow diagram of an example process for generating a molecule embedding using a graph neural network.
FIG. 7B illustrates an example of generating a molecule embedding using a graph neural network.
FIG. 8 is a flow diagram of an example process for training a property prediction machine learning model.

Like reference numbers and designations in the various drawings indicate like elements.

### DETAILED DESCRIPTION

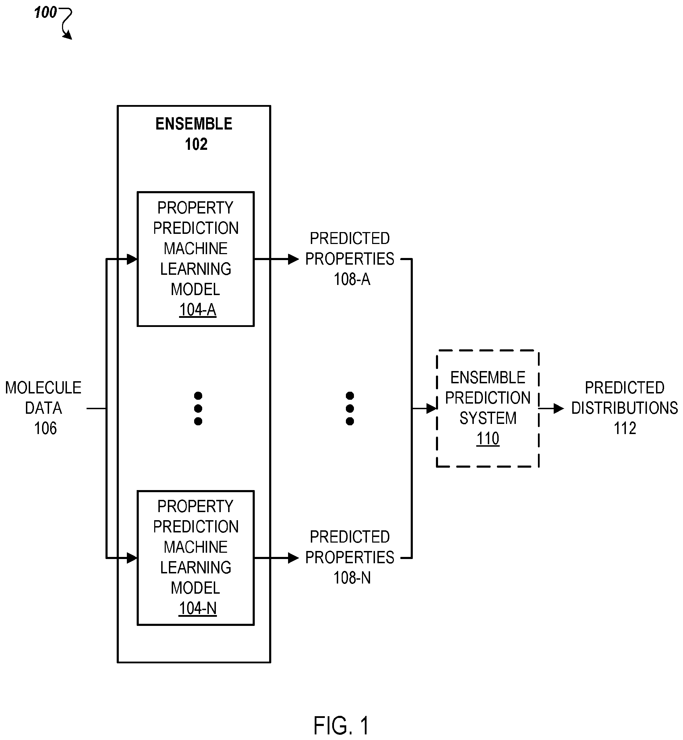

FIG. 1 shows an example molecule property prediction system 100. The molecule property prediction system 100 is an example of a system implemented as computer programs on one or more computers in one or more locations in which the systems, components, and techniques described below are implemented.

The molecule property prediction system 100 includes one or more property prediction machine learning models 104-A through 104-N. In particular, the system 100 can include an ensemble 102 of multiple property prediction machine learning models 104-A through 104-N, e.g., 2, or 5, or 10, or 100, or 1000 property prediction machine learning models. The property prediction machine learning models 104-A through 104-N can each process input molecule data 106 characterizing a molecule to generate predicted molecule properties 108-A through 108-N for the molecule.

In general, the input molecule data 106 can characterize the molecule by specifying a chemical composition and structure for the molecule. The input molecule data 106 can, for example, be a Simplified Molecular-Input Line-Entry System (SMILES) string specifying the molecule, as described by David Weininger in "SMILES, a Chemical Language and Information System. 1. Introduction to Methodology and Encoding Rules" Journal of Chemical Information and Computer Sciences, 28(1) (1988). As another example, the molecule can be a protein and the input molecule data 106 specify, e.g., an amino acid sequence for the protein, a 3D structure of the protein, and so on. As another example, the molecule can be a nucleic acid (e.g., a DNA or an RNA molecule) and the input molecule data 106 specify, e.g., a nucleotide sequence for the nucleic acid, a 3D structure of the nucleic acid, and so on.

Each of the property prediction machine learning models 104-A through 104-N can predict values for each of a same set of molecule properties for the molecule. The set of molecule properties can include a variety of molecular properties for the molecule. For example, the set of molecule properties can include an energy (e.g., a solvation energy) of the molecule. As another example, the set of molecule properties can include predict an acidity (e.g., a pH) of the molecule. As a further example, the set of molecule properties can include a classification of the molecule as being acidic or basic. As yet another example, the set of molecule properties can include solubility properties for the molecule, such as a partition coefficient (*log P*) of the molecule, a distribution coefficient (*log D*) of the molecule, and so on. As another example, the set of molecule properties can include absorption properties, e.g., that characterize permeability and bioavailability of the molecule, e.g., as a score that measures a capacity of the molecule to permeate through the intestinal epithelial barrier or the blood-brain barrier, e.g., as measured using a cell permeability assay. As another example, the set of molecule properties can include distribution properties that characterize how the molecule is distributed in the body once the molecule enters systemic circulation, e.g., as measured using an assay that assesses how strongly the molecule binds to plasma proteins. As another example, the set of molecule properties can include a metabolism property that characterizes how the molecule is metabolized by the body's enzymes (e.g., in the liver), e.g., in terms of the half-life and intrinsic clearance properties of the molecule. As another example, the set of molecule properties can include excretion properties of the molecule, e.g., that characterize the rate and extent of excretion of the molecule in urine or feces. As another example, the set of molecule properties can include toxicity properties of the molecule, e.g., in the form of an IC50 value that specifies a concentration of the molecule that reduces cell viability by 50%.

The molecular properties of the molecule can depend on one another such that determining the value of one molecular property can inform the determination of the value of another molecular property. The value of one molecular property may also be constrained by the value of another property. In order to more accurately predict molecular property values that depend on one another and to more accurately model physical constraints among molecular property values, each of the property prediction machine learning models 104-A through 104-N can generate predicted molecular property values following a hierarchy of the molecular properties.

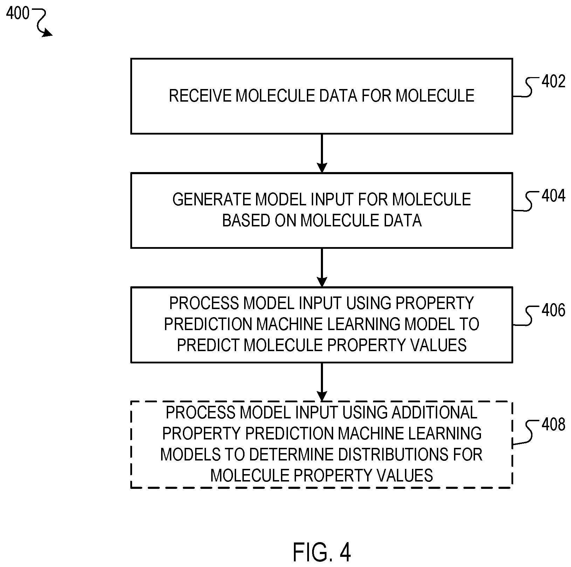

An example process of predicting molecule properties for a molecule using the molecule property prediction system 100 is described in more detail below with reference to FIG. 4.

The property prediction machine learning models 104-A through 104-N can have any architecture suited to generating the predicted molecular property values following a hierarchy of the molecular properties. For example, the property prediction machine learning models 104-A through 104-N can be neural networks configured to generate the predicted molecular property values following the hierarchy of the molecular properties.

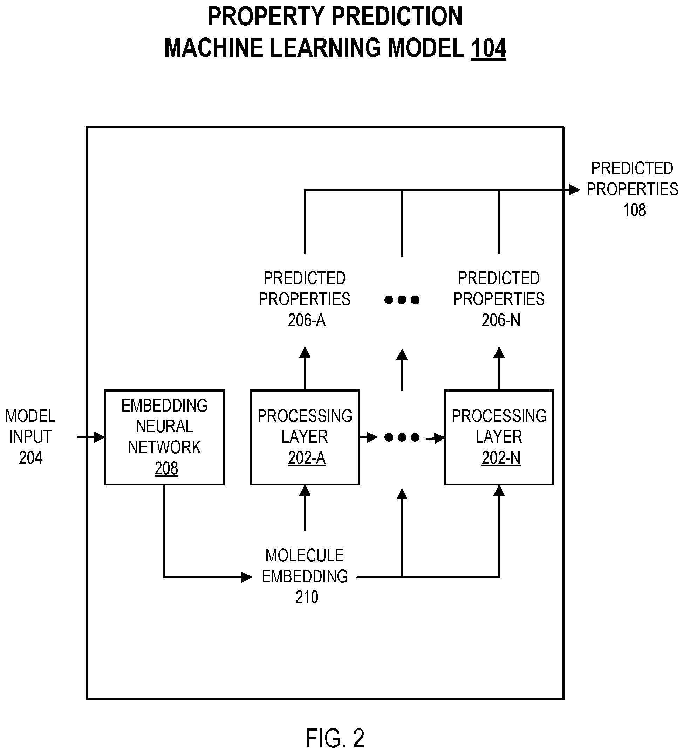

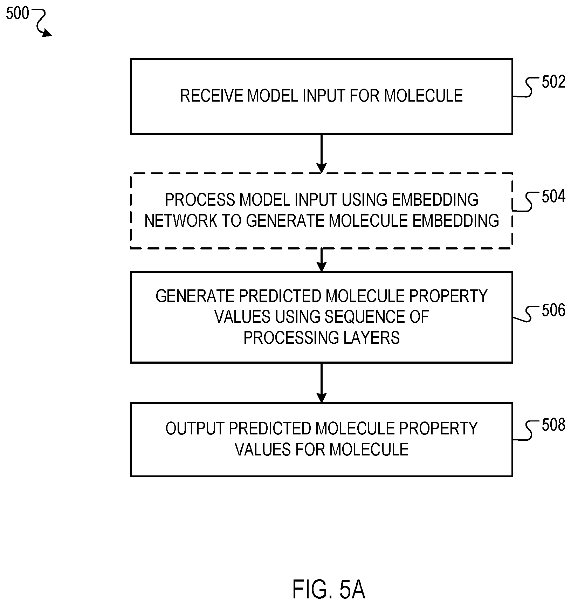

As one example, the hierarchy of the molecular properties can be a pre-defined hierarchy of molecular properties (e.g., as determined by an expert), and the property prediction machine learning models 104-A through 104-N can each include a sequence of processing layers configured to generate predicted molecular property values following the pre-defined hierarchy of the molecular properties. Each of the processing layers can be configured to predict values for a subset (e.g., a proper subset) of molecule properties associated with the processing layer. Each processing layer can predict values for the molecule properties associated with the processing layer based on the model input characterizing the molecule and based on values for molecule properties predicted by preceding processing layers in the sequence of processing layers. An example property prediction machine learning model that can generate predicted molecule property values using a sequence of processing layers is described in more detail below with reference to FIG. 2. An example process for generating predicted molecule property values using a property prediction machine learning model that includes a sequence of processing layers is described in more detail below with reference to FIG. 5A.

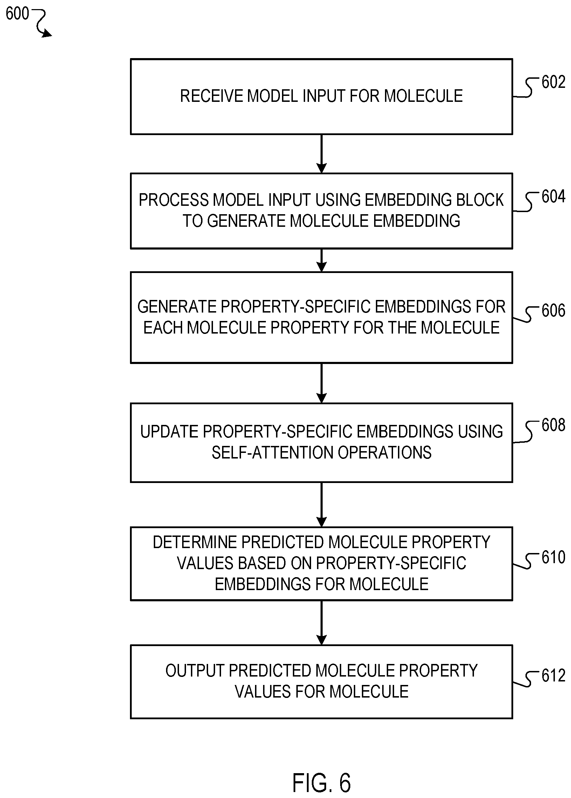

As another example, the property prediction machine learning models 104-A through 104-N can each generate the predicted property values for the molecule using a self-attention mechanism and the hierarchy of the molecular properties can be a machine learned hierarchy of molecular properties. For example, each of the property prediction machine learning models 104-A through 104-N can include one or more self-attention layers that can process and update property-specific embeddings of the predicted molecule properties by performing self-attention operations. An example process for generating predicted molecule property values using a property prediction machine learning model by performing self-attention operations is described in more detail below with reference to FIG. 6.

Each of the property prediction machine learning models 104-A through 104-N can be trained using training data that includes a plurality of training examples, with each training example corresponding to a respective training molecule. Each training example can include a training model input that includes data characterizing the training molecule of the training example and target molecule property values for the training molecule for each of the molecule properties.

The training data can include target molecule property values for molecule properties as determined by physical experiments (e.g., as measured in experimental assays with the training molecules). However, performing physical experiments can be resource intensive and time consuming. Therefore, the system can augment the training data to include target molecule property values determined by other means (i.e., other than physical experiments) in order to increase the amount of training data available to train the property prediction machine learning models 104-A through 104-N. For example, the training data can include target molecule property values for molecule properties as determined by computational simulation (e.g., as determined by molecular dynamics simulations of the training molecules, quantum mechanical simulations of the training molecules, etc.). As another example, the training data can include target molecule property values for molecule properties that can be directly (analytically) calculated based on, e.g., the molecular structure of the training molecules, experimentally or computationally determined properties of the training molecules, and so on. For instance, the training data can include training examples that characterize training molecules and that each include "analytical" target molecule property values including one or more of: molecular weight, number of atoms, number of specific types of bonds (e.g., single bonds, double bonds, and so forth), topological polar surface area (TPSA), and so forth.

The system can thus train each of the property prediction machine learning models to generate predicted molecule property values of: (i) a set of "experimental" molecule properties that are derived from experimental assays, and (ii) a set of "analytical" molecule properties that are computationally determined, e.g., by analytical calculation based on chemical structure or by simulation (as described above). Training the property prediction machine learning models to predict analytical molecule properties can accelerate training of the property prediction machine learning models and can reduce the amount of training data (e.g., experimental data) required for training, e.g., by providing a richer training signal for optimizing the parameter values of the property prediction machine learning models. Training the property prediction machine learning models to predict analytical molecule properties (e.g., that can be derived by direct calculation or simulation) can thus enable reduced consumption of computational resources such as memory and computing power during training.

Each of the property prediction machine learning models 104-A through 104-N can be trained by processing the training model inputs using the property prediction machine learning model to generate predicted molecule property values for the training molecules and updating the property prediction machine learning model using any appropriate machine learning technique to optimize an objective function. The objective function can encourage the property prediction machine learning models 104-A through 104-N to generate predicted molecule property values that accurately predict the target molecule property values of the training data.

In some implementations, the property prediction machine learning models 104-A through 104-N can be trained to generate predicted molecule values that satisfy a chemical constraint for the molecule properties. As one example, the chemical constraint can require that, for a neutral molecule, a logarithm of a partition coefficient (log P) of the molecule is equal to a logarithm of a distribution coefficient (log D) of the molecule at a neutral power of hydrogen (pH). As another example, the chemical constraint can require that, for a charged molecule, a logarithm of a partition coefficient (log P) of the molecule is greater than a logarithm of a distribution coefficient (log D) of the molecule at a neutral power of hydrogen (pH).

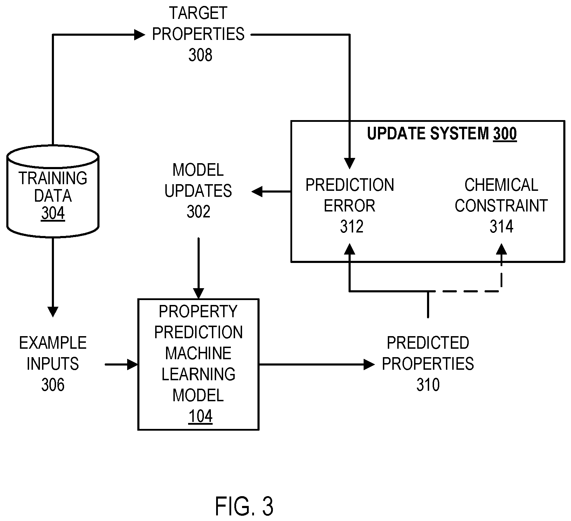

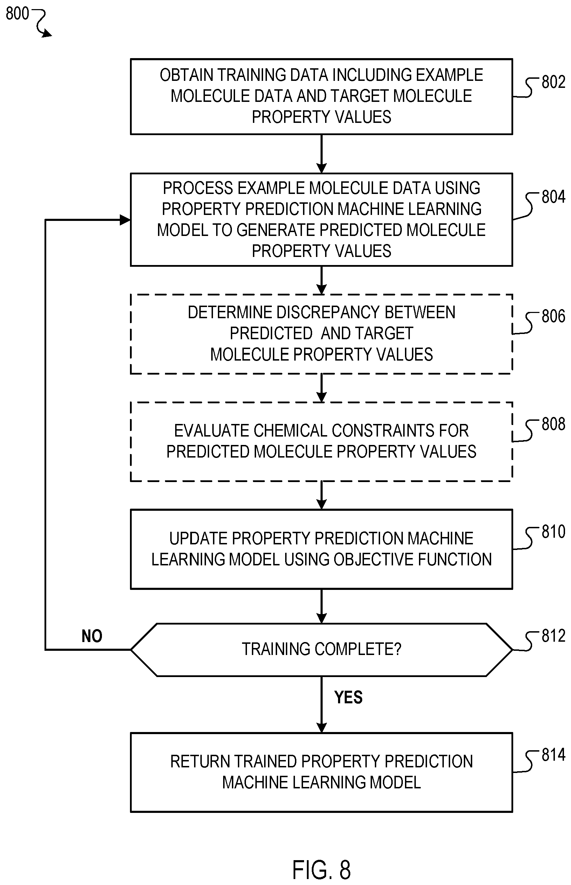

An example process for training the property prediction machine learning models 104-A through 104-N is described in more detail below with reference to FIG. 3 and FIG. 8.

The training data can include training examples of variable data quality (e.g., certainty of target molecule property values). As an example, the training data can include target molecule property values as measured by a plurality of physical and computational experiments, with each experiment having a different level of uncertainty for the measured molecule property values. In some implementations, the training model input for the training example can include data characterizing the data quality of the target molecule property values for the training example. As an example, the data characterizing the data quality of a target molecule property value for a training example can specify a source of the target molecule property value for the training example (where examples of sources can include, e.g., computational simulations carried out using particular methods, or physical experiments carried out using particular methods, and so forth). As another example, the data characterizing the data quality of a target molecule property value for a training example can characterize attributes for a source of the target molecule property value for the training example (e.g., a classification of the data quality from the source, whether the target molecule property value is determined by physical experiment, whether the target molecule property value is obtained from scientific literature, whether the target molecule property value is determined by computational simulation, a type of computational simulation used to determine the target molecule property value, etc.).

During training, the property prediction machine learning models 104-A through 104-N can be trained to generate predicted molecule property values for the training examples as conditioned on the data characterizing the data quality for the training examples. During inference (e.g., after training the property prediction machine learning models 104-A through 104-N), the model input to the property prediction machine learning models 104-A through 104-N can include data characterizing a particular data quality (e.g., a highest data quality). For example, target molecule property values obtained by physical experiment, or a particular type of computational simulation method, may be designated as being of a highest data quality. As another example, each target molecule property value may be assigned a data quality score based on the data characterizing the data quality and, during training, the property prediction machine learning models 104-A through 104-N can be trained to generate predicted molecule property values for the training examples as conditioned on the data quality scores. This can increase the amount of available training data for the property prediction machine learning model, while still enabling the property prediction machine learning model to generate predicted molecule property values characteristic of the highest quality examples available within the training data. That is, the property prediction machine learning model can be trained to take account of differences in data quality when generating the predicted molecule property values.

When the ensemble 102 includes multiple property prediction machine learning models 104-A through 104-N, each of the ensemble of property prediction machine learning models 104-A through 104-N can be trained as described above (e.g., using a different parameter initialization for each model of the ensemble 102, using a different order of training examples for each model of the ensemble 102, etc.). In some implementations, the system 100 can include an ensemble prediction system 110 configured to process the predicted molecule properties 108-A through 108-N generated by the multiple property prediction machine learning models 104-A through 104-N to determine predicted distributions 112 for each of the predicted molecule property values. As an example, the predicted distributions 112 can specify predicted means and variances for each of the predicted molecule property values.

After training the property prediction machine learning models 104-A through 104-N trained, the molecule property prediction system 100 can be used to screen molecules for physical synthesis. For example, the system 100 can receive data identifying a collection of molecules and generate predicted molecule property values for each of the collection of molecules. The system 100 can then select one or more molecules from the collection of molecules for physical synthesis based, at least in part, on the predicted molecule property values. For example, for each molecule in the collection of molecules, the system 100 can evaluate whether the molecule satisfies one or more criteria based on the predicted molecule property values and determining whether to select the molecule for physical synthesis based on whether the molecule satisfies the one or more criteria. The selected molecules can then be physically synthesized for a variety of uses. For example, physical experiments can be performed using a synthesized instance of a selected molecule to experimentally validate values for molecule properties of the selected molecule. Any suitable synthesis techniques can be used to synthesize the selected molecule.

FIG. 2 shows an example property prediction machine learning model 104 that includes a sequence of processing layers 202-A through 202-N.

As described above, the property prediction machine learning model 104 can be configured to process a model input 204 based on molecule data characterizing a molecule and generate predicted molecule properties 108 for the molecule. The property prediction machine learning model 104 can be a neural network (e.g., a property prediction neural network).

As illustrated in FIG. 2, the property prediction machine learning model 104 can include an ordered sequence of processing layers includes a sequence of processing layers 202-A through 202-N configured to hierarchically predict corresponding molecular properties 206-A through 206-N associated with each of the processing layers 202-A through 202-N. In particular, the molecule property prediction machine learning model 104 can be configured to predict a set of multiple molecule properties for the molecule, and each of the processing layers 202-A through 202-N can be associated with a respective subset (e.g., a proper subset) of the set of molecule properties predicted by the model 104.

As an example, each molecule property predicted by the property prediction machine learning model 104 can be associated with exactly one of the processing layers 202-A through 202-N. The property prediction machine learning model 104 can generate predicted values for each of the molecule properties using the prediction layer associated with the molecule property.

Each of the processing layers 202-A through 202-N can process a respective input for the processing layer to predict the molecule properties associated with the processing layer. For example, the input for the first processing layer (e.g., processing layer 202-A) in the sequence of processing layers can include the model input 204 to the property prediction machine learning model 104. The input for each processing layer after the first processing layer can include both the model input 104 to the property prediction machine learning model 104 and predicted molecule property values generated by preceding processing layers in the sequence of processing layers 202-A through 202-N.

Each of the processing layers 202-A through 202-N can have any appropriate architecture suited to generating predicted values for the corresponding predicted molecule properties 206-A through 206-N. As an example, the processing layers 202-A through 202-N can include, e.g., random forest models, support vector machines, and so on. As another example, the processing layers 202-A through 202-N can include neural networks, such as fully connected neural networks, recurrent neural networks, Transformers, and so on.

An example process for generating predicted molecule property values using a property prediction machine learning model that includes a sequence of processing layers is described in more detail below with reference to FIG. 5A.

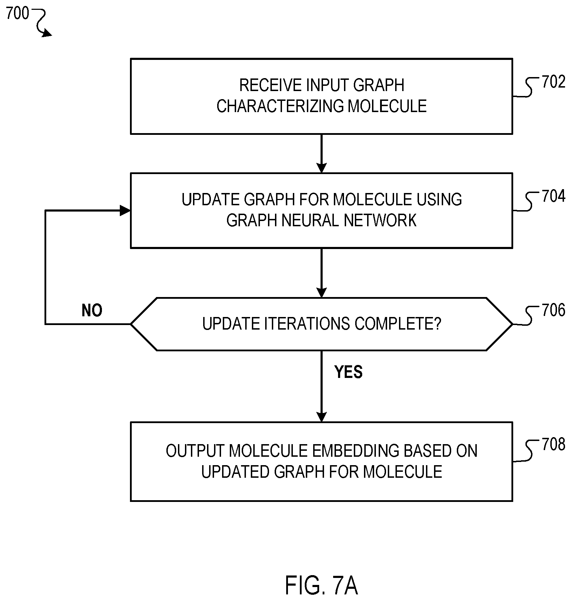

In some implementations, the property prediction machine learning model 104 can include an embedding neural network 208 configured to process the model input 204 for the molecule can generate a molecule embedding 210 for the molecule. The embedding neural network 208 can have any appropriate architecture suited to processing the model input 204. For example, the embedding neural network 208 can be, e.g., a multi-layer perceptron network, a Transformer network, a recurrent neural network, and so on. As another example, the embedding neural network 208 can be a graph neural network and the model input 204 can include a graph characterizing the molecule. For example, the graph characterizing the molecule can include graph nodes characterizing atoms of the molecule and graph edges characterizing chemical bonds between atoms of the molecule. The embedding neural network 208 can process the graph characterizing the molecule to generate the molecule embedding 210. An example process of generating the molecule embedding 210 using by processing the graph characterizing the molecule using a graph neural network is described in more detail below with reference to FIG. 7A.

When the property prediction machine learning model 104 includes an embedding neural network 208, each of the processing layers 202-A through 202-N can be configured to process the molecule embedding 210 for the molecule as part of predicting molecule properties for the molecule. For example, the input for the first processing layer (e.g., processing layer 202-A) in the sequence of processing layers can include the molecule embedding 210. The input for each processing layer after the first processing layer can include both the molecule embedding 210 and predicted molecule property values generated by preceding processing layers in the sequence of processing layers 202-A through 202-N.

FIG. 3 illustrates training a property prediction machine learning model 104 by a molecule property prediction system (e.g., the molecule property prediction system 100 of FIG. 1).

The system can include an update system 300. As part of training the property prediction machine learning model 104, the system can generate model updates 302 for the property prediction machine learning model 104 using the update system 300. For example, the model updates 302 can include updated parameters for the property prediction machine learning model 104.

The system can train the property prediction machine learning model 104 using training data 304 that includes a plurality of training examples, with each training example corresponding to a respective training molecule. Each training example can include an example model input that includes data characterizing the training molecule of the training example and target molecule property values for the training molecule for each of the molecule properties.

In some implementations, the target molecule property values for each training example can be molecule property values for the training molecules under a shared set of experimental conditions (e.g., a temperature range, a pH range, a solvent, etc.). For example, the system can obtain the training data 304 by querying an external database of physical and computational experiment data for training examples obtained under the shared set of experimental conditions. As used herein, experimental conditions can refer to conditions used in physical experiments or computational experiments, e.g., as determined by input parameters of the computational experiments. Training examples are available from publicly or commercially available databases, such as ChEMBL(https://www.ebi.ac.uk/chembll). which is a manually curated database of bioactive molecules with drug-like properties.

As part of training the property prediction machine learning model 104, the system can use the property prediction machine learning model 104 to process example model inputs 306 from the training data 304 to generate corresponding predicted molecule properties 310. The update system 300 can process the predicted properties 310 and corresponding target properties 308 from the training data 304 in order to generate the model updates 302. The update system 300 can generate the model updates 302 to optimize any appropriate objective function using a machine learning training technique.

The objective function can measure a discrepancy between the predicted molecule property values and the target molecule property values for the training examples of the training data 304, e.g., by measuring a prediction error 312 between the predicted molecule properties 310 and the corresponding target properties 308. For example, the objective function can measure, for each numerical molecule property, a regression error (e.g., an L2 norm, an L1 norm, etc.) between the target value and the predicted value for the molecule property. As another example, the objective function can measure, for each categorical molecular property, a classification error (e.g., a cross-entropy loss) between the target value and the predicted value for the molecule property.

In some implementations, the update system 300 can train the property prediction machine learning model 104 to generate predicted molecule property values that satisfy a chemical constraint 314 for the molecule properties. For example, the objective function can include a term that evaluates whether the predicted molecule property values generated by the property prediction machine learning model satisfy the chemical constraint 314 on molecule property values. In general, the chemical constraint 314 can define an allowable region in a joint space of possible values of the plurality of molecule properties and the objective function can penalize predicted molecule property values that are outside the defined allowable region in the joint space of possible molecule property values.

As one example, the chemical constraint 314 can require that, for a neutral molecule, a logarithm of a partition coefficient (log P) of the molecule is equal to a logarithm of a distribution coefficient (log D) of the molecule at a neutral power of hydrogen (pH). As another example, the chemical constraint 314 can require that, for a charged molecule, a logarithm of a partition coefficient (log P) of the molecule is greater than a logarithm of a distribution coefficient (log D) of the molecule at a neutral power of hydrogen (pH).

FIG. 4 is a flow diagram of an example process for predicting molecule property values. For convenience, the process 400 will be described as being performed by a system of one or more computers located in one or more locations. For example, a molecule property prediction system, e.g., the molecule property prediction system 100 of FIG. 1, appropriately programmed in accordance with this specification, can perform the process 400.

As described above, the system can include one or more property prediction machine learning models. For example, the system can include an ensemble of property prediction machine learning models. Each of the property prediction machine learning models can be configured to process model inputs characterizing molecules to generate predicted molecule property values for the molecules. For example, each of the property prediction machine learning models can be trained to process model inputs characterizing molecules to generate predicted molecule property values for the molecules following the example training process described in more detail below with reference to FIG. 8.

The system can receive molecule data for a molecule characterizing the molecule (402). The molecule data can characterize the molecule by specifying a chemical composition and structure for the molecule. For example, the molecule data can be a Simplified Molecular-Input Line-Entry System (SMILES) string specifying the molecule. As another example, the molecule can be a protein and the molecule data specify, e.g., an amino acid sequence for the protein, a 3D structure of the protein, and so on. As another example, the molecule can be a nucleic acid (e.g., a DNA or an RNA molecule) and the molecule data specify, e.g., a nucleotide sequence for the nucleic acid, a 3D structure of the nucleic acid, and so on.

The system can generate a model input for the molecule based on the received molecule data (404). In some implementations, the model input can include a graph characterizing the molecule. For example, the graph characterizing the molecule can include graph nodes characterizing atoms of the molecule and graph edges characterizing chemical bonds between atoms of the molecule.

In some implementations, the model input for the molecule can include data characterizing a particular data quality (e.g., a highest data quality). The system can use the property prediction machine learning model to generate predicted molecule property values characteristic of the particular data quality specified by the model input. As an example, the data characterizing the data quality can specify, for each molecule property, a source of the molecule property value for the molecule. As another example, the data characterizing the data quality can characterize, for each molecule property, attributes for a source of the molecule property value for the molecule (e.g., a classification of the data quality from the source, whether the molecule property value is determined by physical experiment, whether the molecule property value is obtained from scientific literature, whether the molecule property value is determined by computational simulation, a type of computational simulation used to determine the molecule property value, etc.).

In particular, the model input for the molecule can include one or more data quality embeddings that characterize the data quality for the molecule. For example, the data characterizing the data quality can include one-hot embeddings that identify sources of molecule property values for the molecule (e.g., from among a pre-determined set of data sources). As another example, the data characterizing the data quality can include data quality embeddings that specify attributes of sources of molecule property values for the molecule.

The system can then process the model input using one of the property prediction machine learning models of the system to predict molecule properties for the molecule (406). **In** particular, system can process the model input the property prediction machine learning model to generate, for each molecule property in a set of multiple molecule properties, a respective predicted molecule property value of the molecule property for the molecule.

As described above, the property prediction machine learning model can generate the predicted molecule property values for the molecule following a hierarchy of the molecule properties. For example, the property prediction machine learning model can include an ordered sequence of processing layers configured to generate the predicted molecule property values for the molecule following a pre-defined hierarchy of the molecule properties. An example process for predicting molecule property values using a property prediction machine learning model that includes a sequence of processing layers is described in more detail below with reference to FIG. 5A.

As another example, the property prediction machine learning model can process the model input for the molecule using one or more self-attention operations to generate the predicted molecule property values for the molecule following a machine-learned hierarchy of the molecule properties. An example process for predicting molecule property values using a property prediction machine learning model configured to perform one or more self-attention operations is described in more detail below with reference to FIG. 6.

In some implementations, when the system includes an ensemble of multiple property prediction machine learning models, the system can process the model using one or more additional property prediction models from the ensemble in order to determine distributions for the predicted molecule property values (408). In particular, the system can process the model input using the one or more additional property prediction machine learning models from the ensemble of property prediction machine learning models to determine, for each molecule property in the set of multiple molecule properties, a respective distribution of molecule property values of the molecule property for the molecule. For example, the system can generate predicted molecule property values using each of the additional property prediction machine learning models and can determine the distributions for each of the molecule properties based on means and variances of the predicted molecule property values for the molecule generated by the models of the ensemble.

FIG. 5A is a flow diagram of an example process 500 for predicting molecule property values using a property prediction machine learning model that includes an ordered sequence of processing layers. For convenience, the process 500 will be described as being performed by a system of one or more computers located in one or more locations. For example, a molecule property prediction system, e.g., the molecule property prediction system 100 of FIG. 1, appropriately programmed in accordance with this specification, can perform the process 500.

The property prediction machine learning model can receive a model input for a molecule (502). In general, the model input for the molecule can characterize a chemical structure and composition of the molecule. For example, in some implementations, the model input can include a graph characterizing the molecule, which can include graph nodes characterizing atoms of the molecule and graph edges characterizing chemical bonds between atoms of the molecule.

In some implementations, the model input for the molecule can include data characterizing a particular data quality (e.g., a highest data quality). When the model input for the molecule includes data characterizing a particular data quality, the property prediction machine learning model can generate predicted molecule property values characteristic of the particular data quality specified by the model input as conditioned on the data characterizing the particular data quality.

As described above with reference to FIG. 4, the model input for the molecule can include one or more data quality embeddings that characterize the particular data quality for the molecule. During training of the property prediction machine learning model, the data quality embeddings for each molecule can be obtained from the training data for the property prediction machine learning model. Once the property prediction machine learning model has been trained, the model input can include, e.g., data quality embeddings as specified by a user, default data quality embeddings (e.g., default quality embeddings associated with a highest data quality), and so on.

In some implementations, the prediction machine learning model can include an embedding neural network and can process the model input using the embedding neural network to generate a molecule embedding for the molecule (504). The embedding neural network can have any appropriate architecture suited to processing the model input for the molecule. For example, the embedding neural network can include, e.g., a multi-layer perceptron network, a Transformer network, a recurrent neural network, and so on. As another example, when the model input includes a graph characterizing the molecule, the embedding neural network can include a graph neural network and the prediction machine learning model can generate the molecule embedding by processing the graph characterizing the molecule using the graph neural network. When the model input includes data quality embeddings characterizing a data quality for the molecule, the system can include the data quality embeddings within the graph for the molecule (e.g., by including the data quality embeddings within embeddings for graph elements of the graph for the molecule). An example process for generating the molecule embedding using a graph neural network is described in more detail below with reference to FIG. 7A.

The property prediction machine learning model can generate predicted molecule property values for the molecule using the ordered sequence of processing layers (506). In particular, each processing layer in the ordered sequence of processing layers can be associated with a respective proper subset of the set of molecule properties and can generate predicted molecule property values for each of the molecule properties associated with the processing layer. The first processing layer in the ordered sequence of processing layers can generate a respective predicted molecule property value of each molecule property associated with the first processing layer based on the model input to the property prediction machine learning model. Each processing layer after the first processing layer in the ordered sequence of processing layers generates a respective predicted molecule property value of each molecule property associated with the processing layer based on: (i) the model input to the property prediction machine learning model, and (ii) predicted molecule property values generated by one or more preceding processing layers in the sequence of processing layers.

When the property prediction machine learning model generates a molecule embedding for the molecule, each processing layer in the sequence of processing layers can process the molecule embedding as a layer input for the processing layer. For example, the first processing layer in the sequence of processing layers can process a layer input that includes the embedding of the molecule generated by the embedding neural network. Each processing layer after the first processing layer can process a layer input that includes: (i) the embedding of the molecule generated by the embedding neural network, and (ii) the predicted molecule property values generated by the one or more preceding processing layers in the sequence of processing layers. For example, the layer input for each processing layer can be a concatenation of the embedding of the molecule generated by the embedding neural network and the predicted molecule property values generated by the one or more preceding processing layers.

In some implementations, for each molecule property in the set of molecule properties, exactly one processing layer of the ordered sequence of processing layers can be associated with and generate a predicted molecule property value for the molecule property.

The property prediction machine learning model can finally output the predicted molecule property values for the molecule (508).

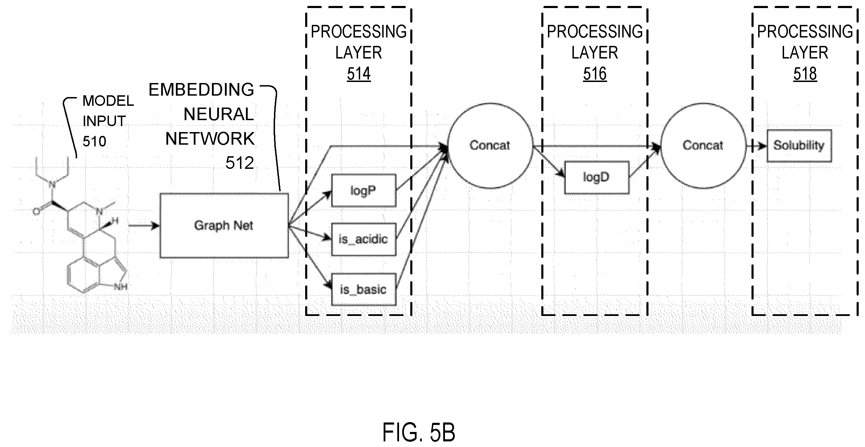

FIG. 5B illustrates an example of predicting molecule property values using a property prediction machine learning model with a sequence of processing layers.

As illustrated, the property prediction machine learning model can process a model input 510 characterizing a molecule to predict values for a set of molecule properties for the molecule. The property prediction machine learning model illustrated in FIG. 5B includes an embedding neural network 512 that can generate a molecule embedding characterizing the molecule based on the model input 510. The property prediction machine learning model illustrated in FIG. 5B includes processing layers 514, 516, and 518, each configured to predict a respective set of molecule properties for the molecule. For example, the processing layer 514 is configured to predict, based on the molecule embedding generated by the embedding neural network 512, a partition coefficient (log P) for the molecule, a classification of whether the molecule is acidic, and a classification of whether the molecule is basic. The processing layer 516 is configured to predict, based on the molecule embedding generated by the embedding neural network 512 and the molecule properties predicted by the preceding processing layer 514, a distribution coefficient (log D) for the molecule. The processing layer 516 is configured to predict, based on the molecule embedding generated by the embedding neural network 512 and the molecule properties predicted by the preceding processing layers 514 and 516, a solubility characteristic for the molecule.

FIG. 6 is a flow diagram of an example process 600 for predicting molecule property values by performing self-attention operations using a property prediction machine learning model implemented as a property prediction neural network. For convenience, the process 600 will be described as being performed by a system of one or more computers located in one or more locations. For example, a molecule property prediction system, e.g., the molecule property prediction system 100 of FIG. 1, appropriately programmed in accordance with this specification, can perform the process 600.

The property prediction machine learning model can receive a model input for a molecule (602). In general, the model input for the molecule can characterize a structure and composition of the molecule. For example, in some implementations, the model input can include a graph characterizing the molecule, which can include graph nodes characterizing atoms of the molecule and graph edges characterizing chemical bonds between atoms of the molecule. As another example, the molecule can be a protein and the graph can characterize the protein, e.g., by including graph nodes characterizing amino acid residues of the protein. As another example, the molecule can be a nucleic acid (e.g., a DNA or an RNA molecule) and the graph can characterize the nucleic acid, e.g., by including graph nodes characterizing nucleotides of the nucleic acid.

In some implementation, the model input for the molecule can include data characterizing a particular data quality (e.g., a highest data quality). When the model input for the molecule includes data characterizing a particular data quality, the property prediction machine learning model can generate predicted molecule property values characteristic of the particular data quality specified by the model input as conditioned on the data characterizing the particular data quality.

As described above with reference to FIG. 4, the model input for the molecule can include one or more data quality embeddings that characterize the particular data quality for the molecule. During training of the property prediction machine learning model, the data quality embeddings for each molecule can be obtained from the training data for the property prediction machine learning model. Once the property prediction machine learning model has been trained, the model input can include, e.g., data quality embeddings as specified by a user, default data quality embeddings (e.g., default quality embeddings associated with a highest data quality), and so on.

The property prediction machine learning model can process the model input using an embedding block to generate a molecule embedding for the molecule (604). The embedding block can have any appropriate architecture suited to processing the model input for the molecule. For example, the embedding block can include, e.g., a multi-layer perceptron network, a Transformer network, a recurrent neural network, and so on. As another example, when the model input includes a graph characterizing the molecule, the embedding block can include a graph neural network and the prediction machine learning model can generate the molecule embedding by processing the graph characterizing the molecule using the graph neural network. When the model input includes data quality embeddings characterizing a data quality for the molecule, the system can include the data quality embeddings within the graph for the molecule (e.g., by including the data quality embeddings within embeddings for graph elements of the graph for the molecule). An example process for generating the molecule embedding using a graph neural network is described in more detail below with reference to FIG. 7A.

The property prediction machine learning model can generate a respective property-specific embedding for each molecule property in a set of molecule properties (606). As one example, the property prediction machine learning model can initialize the property-specific embeddings to pre-determined numerical values (e.g., zeros). As another example, the property prediction machine learning model can initialize the property-specific embeddings to machine learned initial embeddings. As another example, the property prediction machine learning model can include property-specific encoder blocks for each molecule property in the set of molecule properties configured to generate the property-specific embeddings based on the model input for the molecule. For example, when the property prediction machine learning model generates a molecule embedding for the molecule, each of the property-specific encoder blocks can be configured to generate the respective property-specific embedding by processing the molecule embedding.

The property prediction machine learning model can then process the property-specific embeddings to update the property-specific embeddings by performing one or more self-attention operations (608). For example, the property prediction machine learning model can include one or more self-attention layers, each configured to perform a respective self-attention operation for the self-attention layer. Each of the self-attention layers can process the property-specific embeddings of the molecule properties to generate a respective attention weight for each pair of property-specific embeddings of the molecule properties. Each self-attention layer can update the property-specific embeddings of the molecule properties based on the attention weights generated by the self-attention layer. In some implementations, each self-attention layer can be configured to process and update both the property-specific embeddings and the molecule embedding for the molecule by performing the one or more self-attention operations.

The property prediction machine learning model can then determine predicted molecule property values for the molecule based on the updated property-specific embeddings (610). In particular, the property prediction machine learning model can process the property-specific embeddings of the molecule properties to generate the predicted molecule property values for the molecule. As an example, the property prediction machine learning model can include, for each of the set of molecule properties, a property-specific decoder network configured to process the respective updated property-specific embedding to generate the corresponding predicted molecule property value.

The property prediction machine learning model can finally output the predicted molecule property values for the molecule (612).

FIG. 7A is a flow diagram of an example process 700 for generating a molecule embedding using a graph neural network. For convenience, the process 700 will be described as being performed by a system of one or more computers located in one or more locations. For example, a molecule property prediction system, e.g., the molecule property prediction system 100 of FIG. 1, appropriately programmed in accordance with this specification, can perform the process 700.

The graph neural network can receive an input graph characterizing a molecule (702). The graph characterizing the molecule can include a plurality of graph nodes, characterizing a corresponding atom within the molecule. Each graph node can be associated with a respective atom embedding that includes data characterizing properties of the corresponding atom of the molecule, e.g., a chemical element of the corresponding atom, a spatial position of the corresponding atom, a charge of the corresponding atom, and so on. The graph characterizing the molecule can include one or more graph edges, each connecting a respective pair of graph nodes within the graph and characterizing a corresponding chemical bond between a pair of atoms within the molecule. Each graph edge can be associated with a respective bond embedding that includes data characterizing properties of the corresponding chemical bond of the molecule, e.g., a bond distance for the corresponding chemical bond, a bond type for the corresponding chemical bond, and so on.

As described above, the graph for the molecule can include data quality embeddings that characterize a data quality for the molecule. For example, the atom embeddings, the bond embeddings, or can include the data quality embeddings for the molecule.

The graph neural network can update the graph characterizing the molecule over a sequence of update iterations.

At each update iteration, the graph neural network can process the current graph characterizing the molecule as of the update iteration to update the graph characterizing the molecule (704). In some implementations, the graph neural network can include one or more update layers. At each update iteration, the graph neural network can update the embeddings associated with the graph nodes and graph edges by processing the model input using the one or more update layers of the graph neural network.

As an example, the update layers can be message passing layers, each configured to perform a respective message passing operation. The graph neural network can update the embeddings associated with the graph nodes and graph edges by performing the one or more message passing operations of the update layers. For example, the graph neural network can update the embedding associated with each graph node based on the embeddings associated with neighboring graph nodes that are connected to the graph node by respective graph edges and can update the embedding associated with each graph edge based on the embeddings associated with the graph nodes connected by the graph edge.

At each update iteration, the graph neural network can determine whether the update iterations are complete (706). For example, the graph neural network can determine that the update iterations are complete after a pre-determined number of update iterations. If the graph neural network determines that the update iterations are not complete, the graph neural network can continue processing the graph for the molecule for a next update iteration (e.g., return to step 704).

When the graph neural network determines that the update iterations are complete, the graph neural network can generate and output a molecule embedding for the molecule based on the updated graph characterizing the molecule (708). In particular, the graph neural network can generate the embedding of the molecule based on a combination of the updated embeddings associated with the graph nodes. As an example, the graph neural network can generate the embedding of the molecule based on a summation (or average) of the updated embeddings associated with the graph nodes.

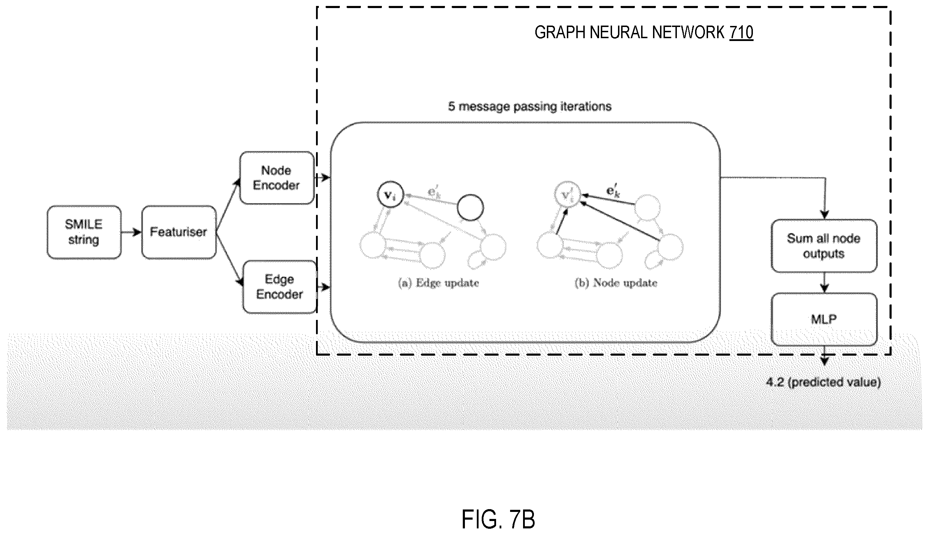

FIG. 7B illustrates an example of generating a molecule embedding for a molecule using a graph neural network 710.

The graph neural network 710 can generate the molecule embedding for the molecule by processing a graph characterizing the molecule. As illustrated in FIG. 7B, the graph characterizing the molecule can be generated based on a SMILE string characterizing molecule, e.g., by including graph nodes characterizing atoms of the molecule and graph edges characterizing chemical bonds between atoms of the molecule.

As illustrated in FIG. 7B, the graph neural network 710 can process the graph characterizing the molecule by performing a sequence of message passing operations (e.g., a sequence of 5 message passing operations), with each message passing operation including, e.g., updating the nodes of the graph and updating the edges of the graph. After performing the message passing operations to update the graph characterizing the molecule, the graph neural network 710 can generate the molecule embedding for the molecule based on the updated graph. For example, as illustrated, the graph neural network 710 can determine a combined node embedding by summing the node embeddings of each node in the updated graph for the molecule and then processing the combined node embedding using a neural network to generate the output molecule embedding for the molecule.

FIG. 8 is a flow diagram of an example process 800 for training a property prediction machine learning model to predict molecule property values for a set of molecule properties. For convenience, the process 800 will be described as being performed by a system of one or more computers located in one or more locations. For example, a molecule property prediction system, e.g., the molecule property prediction system 100 of FIG. 1, appropriately programmed in accordance with this specification, can perform the process 800.

The system can obtain training data that includes example molecule data and target molecule property values for a plurality of training examples (802). Each training example can correspond to a respective training molecule and can include: (i) a training model input that comprises data characterizing the training molecule and (ii) target molecule property values for the training molecule for some or all of the molecule properties in the set of molecule properties. In some implementations, the training model input for each training example includes data characterizing a data quality of the target molecule property values for the training example and the system can train the property prediction machine learning model to generate predicted molecule property values conditioned on the data qualities specified by the model inputs.

For each training example and for each of the set of multiple molecule properties, the target molecule property value for the molecule of the training example can be a molecule property value for the molecule of the training example under a shared set of experimental conditions. For example, the system can obtain the training data by querying an external database containing experimentally and computationally determined molecule property values for target molecule property values obtained under the shared set of experimental conditions.

The system can train the property prediction machine learning model over a sequence of training iterations. In particular, during each training iteration, the system can update the property prediction machine learning model using an objective function for training the property prediction machine learning model that depends on the predicted molecule property values generated by the property prediction machine learning model for the training examples.

At each training iteration, the system can process example molecule data for training examples for the training iteration using the property prediction machine learning model to generate predicted molecule property values for the training molecules of the training examples for the training iteration (804).

In some implementations, the objective function for training the property prediction machine learning model can include a term that measures a discrepancy between predicted molecule property values and corresponding target molecule property values and the system can determine the discrepancy between the predicted and corresponding target molecule property values for the training examples for the training iteration (806). When a training example includes target molecule property values for only some of the set of molecule properties, the system can determine the discrepancy for the target molecule property values included in the training example.

In some implementations, the objective function can include a term that evaluates whether the predicted molecule property values generated by the property prediction machine learning model satisfy a chemical constraint on molecule property values, and the system can evaluate whether the predicted molecule property values for the training examples for the training iteration satisfy the chemical constraint (808). For example, the chemical constraint of molecule property values can define, for a plurality of molecule properties, an allowable region in a joint space of possible values of the plurality of molecule properties. The chemical constraint can penalize predicted molecule property values for the plurality of molecule properties that are outside the allowable region in the joint space of possible values of the plurality of molecule properties.

As one example, the chemical constraint can require that, for a neutral molecule, a logarithm of a partition coefficient (log P) of the molecule is equal to a logarithm of a distribution coefficient (log D) of the molecule at a neutral power of hydrogen (pH). As another example, the chemical constraint can require that, for a charged molecule, a logarithm of a partition coefficient (log P) of the molecule is greater than a logarithm of a distribution coefficient (log D) of the molecule at a neutral power of hydrogen (pH).

The system can then update the property prediction machine learning model using the objective function (810). In particular, the system can update parameters of the property prediction machine learning model using any appropriate machine learning technique to optimize the objective function. For example, the system can determine a gradient of the objective function with respect to the parameters of the property prediction machine learning model and can update the parameters of the property prediction machine learning model based on the gradient using, e.g., stochastic gradient descent, ADAM, and so on. As a further example, when the property prediction machine learning model includes an embedding neural network, the system can backpropagate the gradient of the objective function through portions of the predictive machine learning model (e.g., processing layers, self-attention layers, etc.) into the embedding neural network to update the embedding neural network.

At each training iteration, the system can determine whether the training is complete (812). As an example, the system can determine that training is complete after a pre-determined number of training iterations. As another example, the system can determine that training is complete when a value of the objective function for the training iteration falls below a pre-defined threshold. As another example, the system can determine that training is complete when a different between values of the objective function for the current training iteration and a previous training iteration falls below a pre-defined threshold.

If the system determines that the training is not complete, the system can continue to a next training iteration (e.g., return to step 804).

When the system determines that training is complete, the system can return the trained property prediction machine learning model (814).

In general, the molecule property prediction system 100 and process 400 can be used for obtaining a ligand, in particular a drug, that binds to a target molecule, in particular a drug target such as a protein. For example the target molecule can includes a receptor or enzyme and the ligand can be an agonist or antagonist of the receptor or enzyme. The ligand can be a small molecule (<1000Da) or a biological molecule such as a polypeptide or protein, polynucleotide, or polynucleoside. As another example, the ligand, e.g. the agonist or antagonist, may comprise an antibody or aptamer and the target molecule, e.g. the receptor, may comprise an antibody or aptamer target such as a virus coat protein, or a protein expressed on a cancer cell. For example, the antibody or aptamer may bind to the target and act as an agonist for a particular receptor; alternatively, the antibody or aptamer may prevent binding of another ligand to the target, and hence prevent activation of a relevant biological pathway. One or more ligands can be selected from a plurality of candidate ligands based on molecular properties predicted using the molecule property prediction system 100 or process 400. For example, the candidate ligands can be screened based on predictions of one or more of a partition coefficient, a distribution coefficient, absorption properties, and so on. The selected ligand(s) may be physically synthesized and then, e.g., tested in vitro or in vivo.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and computeintensive parts of machine learning training or production, i.e., inference, workloads.

Machine learning models can be implemented and deployed using a machine learning framework, e.g., a TensorFlow framework, or a Jax framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

Further innovative aspects of the present disclosure are set out in the following numbered clauses, which are not claims:
Clause 1. A method performed by one or more computers, the method comprising:
   receiving molecule data characterizing a molecule; and
   processing a model input based on the molecule data characterizing the molecule using a property prediction machine learning model to generate, for each molecule property in a set of multiple molecule properties, a respective predicted molecule property value of the molecule property for the molecule, wherein:
      the property prediction machine learning model comprises an ordered sequence of processing layers;
      each processing layer in the ordered sequence of processing layers is associated with a respective proper subset of the set of molecule properties; and
      each processing layer after the first processing layer in the ordered sequence of processing layers generates a respective predicted molecule property value of each molecule property associated with the processing layer based on: (i) the model input to the property prediction machine learning model, and (ii) predicted molecule property values generated by one or more preceding processing layers that precede the processing layer in the sequence of processing layers.
Clause 2. The method of clause 1, wherein the first processing layer in the ordered sequence of processing layers generates a respective predicted molecule property value of each molecule property associated with the first processing layer based on the model input to the property prediction machine learning model.
Clause 3. The method of clause 1 or clause 2, wherein for each molecule property in the set of molecule properties, exactly one processing layer of the ordered sequence of processing layers generates a predicted molecule property value for the molecule property.
Clause 4. The method of any preceding clause, wherein the property prediction machine learning model has been trained by operations comprising:
   obtaining training data comprising a plurality of training examples, wherein each training example corresponds to a respective training molecule and includes: (i) a training model input that comprises data characterizing the training molecule, and (ii) a target molecule property value for the training molecule for each of one or more molecule properties of the set of multiple molecule properties; and
   training the property prediction machine learning model on the plurality of training examples using a machine learning technique.
Clause 5. The method of clause 4, wherein training the property prediction machine learning model on the plurality of training examples using the machine learning technique comprises, for each of the plurality of training examples:
   processing the training model input of the training example using the property prediction machine learning model and in accordance with current values of a set of property prediction machine learning model parameters to generate, for each molecule property in the set of multiple molecule properties, a respective predicted molecule property value for the corresponding training molecule; and
   updating the current values of the set of property prediction machine learning model parameters based on an objective function that depends on the predicted molecule property values generated by the property prediction machine learning model for the training example.
Clause 6. The method of clause 5, wherein the objective function includes a term that measures a discrepancy between: (i) the target molecule property values specified by the training example, and (ii) the predicted molecule property values generated by the property prediction machine learning model by processing the training model input of the training example.
Clause 7. The method of clause 5 or clause 6, wherein the objective function includes a term that evaluates whether the predicted molecule property values generated by the property prediction machine learning model satisfy a chemical constraint on molecule property values.
Clause 8. The method of clause 7, wherein the chemical constraint of molecule property values defines, for a plurality of molecule properties, an allowable region in a joint space of possible values of the plurality of molecule properties; and
   the chemical constraint penalizes predicted molecule property values for the plurality of molecule properties that are outside the allowable region in the joint space of possible values of the plurality of molecule properties.
Clause 9. The method of clause 7 or clause 8, wherein the chemical constraint requires that, for a neutral molecule, a logarithm of a partition coefficient (log P) of the molecule is equal to a logarithm of a distribution coefficient (log D) of the molecule at a neutral power of hydrogen (pH).
Clause 10. The method of any one of clauses 7-9, wherein the chemical constraint requires that, for a charged molecule, a logarithm of a partition coefficient (log P) of the molecule is greater than a logarithm of a distribution coefficient (log D) of the molecule at a neutral power of hydrogen (pH).
Clause 11. The method of any one of clauses 4-10, wherein, for each training example and for each of the set of multiple molecule properties, the target molecule property value for the molecule of the training example is a molecule property value for the molecule of the training example under a shared set of experimental conditions.
Clause 12. The method of any preceding clause, wherein processing the model input based on the molecule data characterizing the molecule using the property prediction machine learning model to generate, for each molecule property in a set of multiple molecule properties, a respective predicted molecule property value of the molecule property for the molecule comprises:
   processing the model input based on the molecule data characterizing the molecule using an embedding neural network to generate an embedding of the molecule; and
   wherein for each processing layer after the first processing layer in the ordered sequence of processing layers of the property prediction machine learning model, the processing layer processes a layer input that comprises: (i) the embedding of the molecule generated by the embedding neural network, and (ii) the predicted molecule property values generated by the one or more preceding processing layers that precede the processing layer in the sequence of processing layers.
Clause 13. The method of clause 12, wherein:
   the model input comprises a graph characterizing the molecule; and
   the embedding neural network comprises a graph neural network.
Clause 14. The method of clause 13, wherein the graph characterizing the molecule includes a plurality of graph nodes, wherein each graph node characterizes a corresponding atom within the molecule.
Clause 15. The method of clause 14, wherein each graph node is associated with a respective atom embedding that includes data characterizing properties of the corresponding atom of the molecule.
Clause 16. The method of clause 15, wherein the graph includes one or more graph edges, wherein each graph edge connects a respective pair of graph nodes within the graph and characterizes a corresponding chemical bond between a pair of atoms within the molecule.
Clause 17. The method of clause 16, wherein each graph edge is associated with a respective bond embedding that includes data characterizing properties of the corresponding chemical bond of the molecule.
Clause 18. The method of clause 17, wherein the graph neural network comprises one or more update layers.
Clause 19. The method of clause 18, wherein processing the model input based on the molecule data characterizing the molecule using the embedding neural network to generate the embedding of the molecule:
   for each of one or more update iterations, updating the embeddings associated with the graph nodes and graph edges by processing the model input using the one or more update layers of the graph neural network; and
   generating the embedding of the molecule based on the updated embeddings associated with the graph nodes and graph edges.
Clause 20. The method of clause 19, wherein updating the embeddings associated with the graph nodes and graph edges comprises:
   for each graph node of the graph, updating the embedding associated with the graph node based on the embeddings associated with neighboring graph nodes that are connected to the graph node by respective graph edges; and
   for each graph edge of the graph, updating the embedding associated with the graph edge based on the embeddings associated with the graph nodes connected by the graph edge.
Clause 21. The method of clause 19 or clause 20, wherein generating the embedding of the molecule based on the updated embeddings associated with the graph nodes and graph edges comprises generating the embedding of the molecule based on a combination of the updated embeddings associated with the graph nodes.
Clause 22. The method of clause 21, wherein generating the embedding of the molecule based on a combination of the updated embeddings associated with the graph nodes comprises generating the embedding of the molecule based on a summation of the updated embeddings associated with the graph nodes.
Clause 23. The method of any preceding clause, wherein the set of multiple molecule properties for the molecule includes a partition coefficient (log P) for the molecule.
Clause 24. The method of any preceding clause, wherein the set of multiple molecule properties for the molecule includes a distribution coefficient (log D) for the molecule.
Clause 25. The method of any preceding clause, wherein the set of multiple molecule properties includes a classification of whether the molecule is acidic.
Clause 26. The method of any preceding clause, wherein the set of multiple molecule properties includes a classification of whether the molecule is basic.
Clause 27. The method of any one of clauses 4-26, wherein, for each training example, the training model input for the training example includes data characterizing a data quality of the target molecule property values for the training example.
Clause 28. The method of clause 27, wherein the model input is based on the molecule data characterizing the molecule includes data characterizing a particular data quality.
Clause 29. The method of clause 28, wherein the model input is based on the molecule data characterizing the molecule includes data characterizing a highest data quality.
Clause 30. The method of any preceding clause, wherein the property prediction machine learning model is one of an ensemble of property prediction machine learning models.
Clause 31. The method of clause 30, the method further comprising:
   processing the model input based on the molecule data characterizing the molecule using one or more additional property prediction machine learning models from the ensemble of property prediction machine learning models to determine, for each molecule property in the set of multiple molecule properties, a respective distribution of molecule property values of the molecule property for the molecule.
Clause 32. A method performed by one or more computers, the method comprising:
   receiving molecule data characterizing a molecule;
   processing a model input based on the molecule data characterizing the molecule using a property prediction neural network to generate, for each molecule property in a set of multiple molecule properties, a respective predicted molecule property value of the molecule property for the molecule, comprising:
      processing the model input using a molecule embedding block of the property prediction neural network to generate an embedding of the molecule;
      generating a respective property-specific embedding for each molecule property in the set of multiple molecule properties based on the embedding of the molecule;
      processing the property-specific embeddings of the molecule properties in the set of molecule properties by applying one or more self-attention operations to update the property-specific embeddings of the molecule properties; and
      after applying the self-attention operations to the property-specific embeddings of the molecule properties, processing the property-specific embeddings of the molecule properties to generate the predicted molecule property values.
Clause 33. The method of clause 32, wherein:
   the property prediction neural network comprises one or more self-attention layers; and
   processing the property-specific embeddings of the molecule properties in the set of molecule properties by applying one or more self-attention operations to update the property-specific embeddings of the molecule properties comprises, for each of the one or more self-attention layers of the property prediction neural network:
      processing, by the self-attention layer, the property-specific embeddings of the molecule properties to generate a respective attention weight for each pair of property-specific embeddings of the molecule properties; and
      updating, by the self-attention layer, the property-specific embeddings of the molecule properties based on the attention weights generated by the self-attention layer.
Clause 34. The method of clause 32 or clause 33, wherein the property prediction neural network has been trained by operations comprising:
   obtaining training data comprising a plurality of training examples, wherein each training example corresponds to a respective training molecule and includes: (i) a training model input that comprises data characterizing the training molecule, and (ii) a target molecule property value for the training molecule for each of one or more molecule properties of the set of multiple molecule properties; and
   training the property prediction neural network on the plurality of training examples using a machine learning technique.
Clause 35. The method of clause 34, wherein training the property prediction neural network on the plurality of training examples using the machine learning technique comprises, for each of the plurality of training examples:
   processing the training model input of the training example using the property prediction neural network and in accordance with current values of a set of property prediction neural network parameters to generate, for each molecule property in the set of multiple molecule properties, a respective predicted molecule property value for the corresponding training molecule; and
   updating the current values of the set of property prediction neural network parameters based on an objective function that depends on the predicted molecule property values generated by the property prediction neural network for the training example.
Clause 36. The method of clause 35, wherein the objective function includes a term that measures a discrepancy between: (i) the target molecule property values specified by the training example, and (ii) the predicted molecule property values generated by the property prediction neural network by processing the training model input of the training example.
Clause 37. The method of clause 35 or clause 36, wherein the objective function includes a term that evaluates whether the predicted molecule property values generated by the property prediction neural network satisfy a chemical constraint on molecule property values.
Clause 38. The method of clause 37, wherein the chemical constraint of molecule property values defines, for a plurality of molecule properties, an allowable region in a joint space of possible values of the plurality of molecule properties; and
   the chemical constraint penalizes a predicted molecule property values for the plurality of molecule properties that are outside the allowable region in the joint space of possible values of the plurality of molecule properties.
Clause 39. The method of clause 37 or clause 38, wherein the chemical constraint requires that, for a neutral molecule, a logarithm of a partition coefficient (log P) of the molecule is equal to a logarithm of a distribution coefficient (log D) of the molecule at a neutral power of hydrogen (pH).
Clause 40. The method of any one of clauses 37-39, wherein the chemical constraint requires that, for a charged molecule, a logarithm of a partition coefficient (log P) of the molecule is greater than a logarithm of a distribution coefficient (log D) of the molecule at a neutral power of hydrogen (pH).
Clause 41. The method of any one of clauses 34-40, wherein, for each training example and for each of the set of multiple molecule properties, the target molecule property value for the molecule of the training example is a molecule property value for the molecule of the training example under a shared set of experimental conditions.
Clause 42. The method of one of clauses 32-41, wherein:
   the model input comprises a graph characterizing the molecule; and
   the molecule embedding block of the property prediction neural network comprises a graph neural network.
Clause 43. The method of clause 42, wherein the graph characterizing the molecule includes a plurality of graph nodes, wherein each graph node characterizes a corresponding atom within the molecule.
Clause 44. The method of clause 43, wherein each graph node is associated with a respective atom embedding that includes data characterizing properties of the corresponding atom of the molecule.
Clause 45. The method of clause 44, wherein the graph includes one or more graph edges, wherein each graph edge connects a respective pair of graph nodes within the graph and characterizes a corresponding chemical bond between a pair of atoms within the molecule.
Clause 46. The method of clause 45, wherein each graph edge is associated with a respective bond embedding that includes data characterizing properties of the corresponding chemical bond of the molecule.
Clause 47. The method of clause 46, wherein the graph neural network comprises one or more update layers.
Clause 48. The method of clause 47, wherein processing the model input using the molecule embedding block of the property prediction neural network to generate the embedding of the molecule comprises:
   for each of one or more update iterations, updating the embeddings associated with the graph nodes and graph edges by processing the model input using the one or more update layers of the graph neural network; and
   generating the embedding of the molecule based on the updated embeddings associated with the graph nodes and graph edges.
Clause 49. The method of clause 48, wherein updating the embeddings associated with the graph nodes and graph edges comprises:
   for each graph node of the graph, updating the embedding associated with the graph node based on the embeddings associated with neighboring graph nodes that are connected to the graph node by respective graph edges; and
   for each graph edge of the graph, updating the embedding associated with the graph edge based on the embeddings associated with the graph nodes connected by the graph edge.
Clause 50. The method of clause 48 or clause 49, wherein generating the embedding of the molecule based on the updated embeddings associated with the graph nodes and graph edges comprises generating the embedding of the molecule based on a combination of the updated embeddings associated with the graph nodes.
Clause 51. The method of clause 50, wherein generating the embedding of the molecule based on a combination of the updated embeddings associated with the graph nodes comprises generating the embedding of the molecule based on a summation of the updated embeddings associated with the graph nodes.
Clause 52. The method of any one of clauses 32-51, wherein the set of multiple molecule properties for the molecule includes a partition coefficient (log P) for the molecule.
Clause 53. The method of any one of clauses 32-52, wherein the set of multiple molecule properties for the molecule includes a distribution coefficient (log D) for the molecule.
Clause 54. The method of any one of clauses 32-53, wherein the set of multiple molecule properties includes a classification of whether the molecule is acidic.
Clause 55. The method of any one of clauses 32-54, wherein the set of multiple molecule properties includes a classification of whether the molecule is basic.
Clause 56. The method of any one of clauses 34-55, wherein, for each training example, the training model input for the training example includes data characterizing a data quality of the target molecule property values for the training example.
Clause 57. The method of clause 56, wherein the model input is based on the molecule data characterizing the molecule includes data characterizing a particular data quality.
Clause 58. The method of clause 57, wherein the model input is based on the molecule data characterizing the molecule includes data characterizing a highest data quality.
Clause 59. The method of any one of clauses 32-58, wherein the property prediction neural network is one of an ensemble of property prediction neural networks.
Clause 60. The method of clause 59, the method further comprising:
   processing the model input based on the molecule data characterizing the molecule using one or more additional property prediction neural networks from the ensemble of property prediction neural networks to determine, for each molecule property in the set of multiple molecule properties, a respective distribution of molecule property values of the molecule property for the molecule.
Clause 61. A method, comprising:
   receiving data identifying a collection of molecules;
   generating, for each molecule in the collection of molecules, predicted molecule property values for each of a set of multiple molecule properties of the molecule using the method of any one of clauses 1-60; and
   selecting one or more molecules in the collection of molecules for physical synthesis based, at least in part, on the predicted molecule property values.
Clause 62. The method of clause 61, wherein selecting the one or more molecules in the collection of molecules for physical synthesis based, at least in part, on the predicted molecule property values comprises, for each molecule in the collection of molecules:
   evaluating whether the molecule satisfies one or more criteria based on the predicted molecule property values; and
   determining whether to select the molecule for physical synthesis based on whether the molecule satisfies the one or more criteria based on the predicted molecule property values.
Clause 63. The method of clause 60 or clause 61, further comprising:
   physically synthesizing one or more of the molecules selected for physical synthesis.
Clause 64. The method of any one of clauses 61-63, further comprising, for each of one or more of the molecules selected for physical synthesis:
   performing a physical experiment using a synthesized instance of the molecule to determine experimental values for each of one or more experimentally validated molecule properties of the molecule.
Clause 65. The method of clause 64, wherein the one or more experimentally validated molecule properties includes a partition coefficient (log P) for the molecule.
Clause 66. The method of clause 64 or clause 65, wherein the one or more experimentally validated molecule properties for the molecule includes a distribution coefficient (log D) for the molecule.
Clause 67. The method of any one of clauses 64-66, wherein the one or more experimentally validated molecule properties includes a classification of whether the molecule is acidic.
Clause 68. The method of any one of clauses 64-67, wherein the one or more experimentally validated molecule properties includes a classification of whether the molecule is basic.
Clause 69. A system comprising:
   one or more computers; and
   one or more storage devices communicatively coupled to the one or more computers, wherein the one or more storage devices store instructions that, when executed by the one or more computers, cause the one or more computers to perform the operations of any one of clauses 1-62.
Clause 70. One or more non-transitory computer storage media storing instructions that when executed by one or more computers cause the one or more computers to perform the operations of any one of clauses 1-62.

## Claims

1. A method performed by one or more computers, the method comprising:
receiving molecule data characterizing a molecule; and
processing a model input based on the molecule data characterizing the molecule using a property prediction machine learning model to generate, for each molecule property in a set of multiple molecule properties, a respective predicted molecule property value of the molecule property for the molecule, wherein:
the property prediction machine learning model comprises an ordered sequence of processing layers;
each processing layer in the ordered sequence of processing layers is associated with a respective proper subset of the set of molecule properties; and
each processing layer after the first processing layer in the ordered sequence of processing layers generates a respective predicted molecule property value of each molecule property associated with the processing layer based on: (i) the model input to the property prediction machine learning model, and (ii) predicted molecule property values generated by one or more preceding processing layers that precede the processing layer in the sequence of processing layers.

2. The method of claim 1, wherein:
(i) the first processing layer in the ordered sequence of processing layers generates a respective predicted molecule property value of each molecule property associated with the first processing layer based on the model input to the property prediction machine learning model; and/or
(ii) for each molecule property in the set of molecule properties, exactly one processing layer of the ordered sequence of processing layers generates a predicted molecule property value for the molecule property.

3. A method performed by one or more computers, the method comprising:
receiving molecule data characterizing a molecule;
processing a model input based on the molecule data characterizing the molecule using a property prediction neural network to generate, for each molecule property in a set of multiple molecule properties, a respective predicted molecule property value of the molecule property for the molecule, comprising:
processing the model input using a molecule embedding block of the property prediction neural network to generate an embedding of the molecule;
generating a respective property-specific embedding for each molecule property in the set of multiple molecule properties based on the embedding of the molecule;
processing the property-specific embeddings of the molecule properties in the set of molecule properties by applying one or more self-attention operations to update the property-specific embeddings of the molecule properties; and
after applying the self-attention operations to the property-specific embeddings of the molecule properties, processing the property-specific embeddings of the molecule properties to generate the predicted molecule property values.

4. The method of claim 3, wherein:
the property prediction neural network comprises one or more self-attention layers; and
processing the property-specific embeddings of the molecule properties in the set of molecule properties by applying one or more self-attention operations to update the property-specific embeddings of the molecule properties comprises, for each of the one or more self-attention layers of the property prediction neural network:
processing, by the self-attention layer, the property-specific embeddings of the molecule properties to generate a respective attention weight for each pair of property-specific embeddings of the molecule properties; and
updating, by the self-attention layer, the property-specific embeddings of the molecule properties based on the attention weights generated by the self-attention layer.

5. The method of any preceding claim, wherein the property prediction machine learning model has been trained by operations comprising:
obtaining training data comprising a plurality of training examples, wherein each training example corresponds to a respective training molecule and includes: (i) a training model input that comprises data characterizing the training molecule, and (ii) a target molecule property value for the training molecule for each of one or more molecule properties of the set of multiple molecule properties; and
training the property prediction machine learning model on the plurality of training examples using a machine learning technique.

6. The method of claim 5, wherein training the property prediction machine learning model on the plurality of training examples using the machine learning technique comprises, for each of the plurality of training examples:
processing the training model input of the training example using the property prediction machine learning model and in accordance with current values of a set of property prediction machine learning model parameters to generate, for each molecule property in the set of multiple molecule properties, a respective predicted molecule property value for the corresponding training molecule; and
updating the current values of the set of property prediction machine learning model parameters based on an objective function that depends on the predicted molecule property values generated by the property prediction machine learning model for the training example, and
optionally, wherein the objective function includes a term that measures a discrepancy between: (i) the target molecule property values specified by the training example, and (ii) the predicted molecule property values generated by the property prediction machine learning model by processing the training model input of the training example.

7. The method of claim 6, wherein the objective function includes a term that evaluates whether the predicted molecule property values generated by the property prediction machine learning model satisfy a chemical constraint on molecule property values, and
optionally:
wherein the chemical constraint of molecule property values defines, for a plurality of molecule properties, an allowable region in a joint space of possible values of the plurality of molecule properties; and
the chemical constraint penalizes predicted molecule property values for the plurality of molecule properties that are outside the allowable region in the joint space of possible values of the plurality of molecule properties.

8. The method of claim 7, wherein the chemical constraint requires that:
(i) for a neutral molecule, a logarithm of a partition coefficient, log P, of the molecule is equal to a logarithm of a distribution coefficient, log D, of the molecule at a neutral power of hydrogen, pH; and/or
(ii) for a charged molecule, a logarithm of a partition coefficient, log P, of the molecule is greater than a logarithm of a distribution coefficient, log D, of the molecule at a neutral power of hydrogen, pH.

9. The method of any one of claims 5-8, wherein, for each training example and for each of the set of multiple molecule properties, the target molecule property value for the molecule of the training example is a molecule property value for the molecule of the training example under a shared set of experimental conditions.

10. The method of any preceding claim, when dependent on claim 1, wherein processing the model input based on the molecule data characterizing the molecule using the property prediction machine learning model to generate, for each molecule property in a set of multiple molecule properties, a respective predicted molecule property value of the molecule property for the molecule comprises:
processing the model input based on the molecule data characterizing the molecule using an embedding neural network to generate an embedding of the molecule; and
wherein for each processing layer after the first processing layer in the ordered sequence of processing layers of the property prediction machine learning model, the processing layer processes a layer input that comprises: (i) the embedding of the molecule generated by the embedding neural network, and (ii) the predicted molecule property values generated by the one or more preceding processing layers that precede the processing layer in the sequence of processing layers; and
optionally, wherein:
the model input comprises a graph characterizing the molecule; and
the embedding neural network comprises a graph neural network.

11. The method of any one of claims 3-9 when dependent on claim 3, wherein:
the model input comprises a graph characterizing the molecule; and
the molecule embedding block of the property prediction neural network comprises a graph neural network.

12. The method of claim 10 or 11, wherein the graph characterizing the molecule includes a plurality of graph nodes, wherein:
each graph node characterizes a corresponding atom within the molecule; and
preferably, each graph node is associated with a respective atom embedding that includes data characterizing properties of the corresponding atom of the molecule.

13. The method of claim 12, wherein the graph includes one or more graph edges, wherein each graph edge connects a respective pair of graph nodes within the graph and characterizes a corresponding chemical bond between a pair of atoms within the molecule and
preferably, each graph edge is associated with a respective bond embedding that includes data characterizing properties of the corresponding chemical bond of the molecule; and
more preferably, the graph neural network comprises one or more update layers.

14. The method of claim 13 when dependent on claim 10, wherein processing the model input based on the molecule data characterizing the molecule using the embedding neural network to generate the embedding of the molecule:
for each of one or more update iterations, updating the embeddings associated with the graph nodes and graph edges by processing the model input using the one or more update layers of the graph neural network; and
generating the embedding of the molecule based on the updated embeddings associated with the graph nodes and graph edges.

15. The method of claim 13 when dependent on claim 3, wherein processing the model input using the molecule embedding block of the property prediction neural network to generate the embedding of the molecule comprises:
for each of one or more update iterations, updating the embeddings associated with the graph nodes and graph edges by processing the model input using the one or more update layers of the graph neural network; and
generating the embedding of the molecule based on the updated embeddings associated with the graph nodes and graph edges.

16. The method of claim 14 or claim 15, wherein updating the embeddings associated with the graph nodes and graph edges comprises:
for each graph node of the graph, updating the embedding associated with the graph node based on the embeddings associated with neighboring graph nodes that are connected to the graph node by respective graph edges; and
for each graph edge of the graph, updating the embedding associated with the graph edge based on the embeddings associated with the graph nodes connected by the graph edge, and optionally, wherein:
(i) generating the embedding of the molecule based on the updated embeddings associated with the graph nodes and graph edges comprises generating the embedding of the molecule based on a combination of the updated embeddings associated with the graph nodes;
and
optionally, (ii) generating the embedding of the molecule based on a combination of the updated embeddings associated with the graph nodes comprises generating the embedding of the molecule based on a summation of the updated embeddings associated with the graph nodes.

17. The method of any preceding claim, wherein:
(i) the set of multiple molecule properties for the molecule includes one or more of:
a partition coefficient (log P) for the molecule;
a distribution coefficient (log D) for the molecule;
a classification of whether the molecule is acidic; and
a classification of whether the molecule is basic, and/or
(ii) the property prediction machine learning model is one of an ensemble of property prediction machine learning models, and
optionally, the method further comprises:
processing the model input is based on the molecule data characterizing the molecule using one or more additional property prediction machine learning models from the ensemble of property prediction machine learning models to determine, for each molecule property in the set of multiple molecule properties, a respective distribution of molecule property values of the molecule property for the molecule; and/or
(iii) wherein the property prediction machine learning model is one of an ensemble of property prediction machine learning models, and
optionally, the method further comprises:
processing the model input is based on the molecule data characterizing the molecule using one or more additional property prediction machine learning models from the ensemble of property prediction machine learning models to determine, for each molecule property in the set of multiple molecule properties, a respective distribution of molecule property values of the molecule property for the molecule.

18. The method of any preceding claim when dependent on claim 5, wherein, for each training example, the training model input for the training example includes data characterizing:
a data quality of the target molecule property values for the training example; or
a particular data quality; or
a highest data quality.

19. A method, comprising:
receiving data identifying a collection of molecules;
generating, for each molecule in the collection of molecules, predicted molecule property values for each of a set of multiple molecule properties of the molecule using the method of any one of claims 1-18; and
selecting one or more molecules in the collection of molecules for physical synthesis based, at least in part, on the predicted molecule property values, and
optionally, wherein selecting the one or more molecules in the collection of molecules for physical synthesis based, at least in part, on the predicted molecule property values comprises, for each molecule in the collection of molecules:
evaluating whether the molecule satisfies one or more criteria based on the predicted molecule property values; and
determining whether to select the molecule for physical synthesis based on whether the molecule satisfies the one or more criteria based on the predicted molecule property values.

20. The method of claim 19, further comprising:
physically synthesizing one or more of the molecules selected for physical synthesis; and/or
performing a physical experiment using a synthesized instance of the molecule to determine experimental values for each of one or more experimentally validated molecule properties of the molecule, wherein optionally, the one or more experimentally validated molecule properties includes one or more of:
a partition coefficient (log P) for the molecule;
a distribution coefficient (log D) for the molecule;
a classification of whether the molecule is acidic; and
a classification of whether the molecule is basic.

21. A system comprising:
one or more computers; and
one or more storage devices communicatively coupled to the one or more computers,
wherein the one or more storage devices store instructions that, when executed by the one or more computers, cause the one or more computers to perform the operations of any one of claims 1-19.

22. One or more non-transitory computer storage media storing instructions that when executed by one or more computers cause the one or more computers to perform the operations of any one of claims 1-19.
